# FASCICULE DE BREVET EUROPEEN

(11) **EP 2 021 038 B1**
(45) Date de publication et mention de la délivrance du brevet: **24.03.2010**
(21) Numéro de dépôt: 07766008.2
(22) Date de dépôt: 07.05.2007
(51) Int. Cl.: A61L 2/20

(54) **DISPOSITIF POUR LA DESINFECTION DE LIT**
BETTDESINFEKTIONSVORRICHTUNG
BED DISINFECTING DEVICE

(30) Priorité: 11.05.2006 FR 0651684
(43) Date de publication de la demande: 11.02.2009
(73) Titulaire: Delcher, Bernard, 66000 Perpignan (FR)
(72) Inventeur: Delcher, Bernard, 66000 Perpignan (FR)
(74) Mandataire: Rhein, Alain
(86) Numéro de dépôt international: PCT/FR2007/051228
(87) Numéro de publication internationale: WO 2007/132110

(56) Documents cités:
- WO-A-01/30400
- DE-A1- 3 317 300
- FR-A- 2 773 490
- US-A- 4 937 046
- US-A- 5 713 137

## Description

La présente invention a pour objet un dispositif pour la désinfection de lit, et plus particulièrement pour la désinfection des matelas et des oreillers.

Si dans le domaine hospitalier les lits et la literie font l'objet de soins particuliers, il n'en est pas de même dans le domaine de l'hôtellerie.

En effet, dans les hôtels et analogues, les draps et taies d'oreillers sont changées pour chaque nouveau client, mais les matelas et les oreillers ne sont pas nettoyés, ce qui peut poser des problèmes du point de vue hygiène.

Certains établissements réalisent périodiquement une désinfection des matelas au travers d'une projection de vapeur. Cette méthode n'est pas infaillible puisque le traitement est réalisé manuellement, en sorte que certaines zones peuvent être oubliées, et elle reste coûteuse en terme de main d'oeuvre.

On connaît par ailleurs des dispositifs pour désinfecter des objets volumineux, comme par exemple celui décrit dans le document FR 2 773 490, destiné au traitement d'objets tels que des lits d'hôpitaux, des fauteuils roulants, des meubles ou des chariots d'hôpitaux, et qui comprend une enceinte propre à recevoir ledit objet, dans laquelle un liquide nettoyant ou désinfectant peut être projeté sur ledit objet. Cette enceinte est constituée de panneaux maintenus par une armature faite de l'assemblage de tuyaux qui permettent de véhiculer le produit désinfectant jusqu'à des buses de pulvérisation.

Un tel dispositif, du fait de son encombrement important, est destiné uniquement à un usage hospitalier, et ne peut en aucun cas être utilisé dans le domaine de l'hôtellerie.

On connaît aussi pat le document WO 01/30400 un procédé et un appareil pour stériliser et désinfecter une literie, par exemple un matelas de lit, un couvre-lit, un matelas oriental, un oreiller, etc. au moyen de rayons ultraviolets et d'ozone, ledit appareil étant de type pliable de façon à être plié en position de rangement et déplié pour son utilisation. L'utilisation d'un traitement à l'ozone nécessite d'enfermer le lit et la literie dans une enceinte relativement étanche, tandis que l'utilisation de rayons ultra violets nécessite d'avoir à utiliser des moyens réfléchissants afin de pouvoir atteindre toutes les surfaces à traiter. A cet effet le dispositif objet du document WO 01/30400 comprend une enveloppe destinée à constituée une enceinte dans laquelle sont disposés les objets à traiter, cette enveloppe est prévue étanche, et associée à des moyens pour maintenir le dispositif à une certaine distance du matelas à traiter, manuellement ou au travers de pieds télescopiques, et/ou des moyens de réflexion selon le mode de fonctionnement considéré.

Ce dispositif présente des nombreux inconvénients, outre le fait que l'enveloppe utilisée est d'une conception particulière, et doit présenter des dimensions permettant de faire face à tous les cas de figure, le dispositif est de conception complexe, donc onéreuse, et d'un maniement peu aisé.

On connaît également par le document US 5 713 137 un dispositif comprenant une enceinte destinée à recevoir des objets à traiter, et un moyen de diffusion à l'intérieur de ladite enceinte d'un produit de traitement. Un tel dispositif peut permettre de traiter des oreillers et de la literie, mais ne permet pas de traiter les lits d'un hôtel par exemple, car cela nécessiterait de pouvoir introduire un tel lits dans ladite enceinte qui, si elle était suffisamment grande pour permettre une telle introduction, ne serait pas assez maniable pour la déplacer de chambre en chambre.

La présente invention a pour but de proposer un dispositif pour la désinfection de lit, de conception et d'utilisation simple, permettant de pallier les inconvénients précités.

Le dispositif pour la désinfection de lit selon l'invention comprend un dispositif selon la revendication 1.

Selon une caractéristique additionnelle du dispositif pour la désinfection de lit selon l'invention, le moyen de diffusion consiste en un moyen de vaporisation.

Selon une autre caractéristique additionnelle du dispositif pour la désinfection de lit selon l'invention, le moyen de diffusion comporte des moyens aptes à permettre sa solidarisation à la housse dans la région centrale de celle-ci.

Selon une construction particulière du mode de réalisation préférentiel du dispositif pour la désinfection de lit selon l'invention, le moyen de diffusion est de forme cylindrique et présente extérieurement, dans une partie intermédiaire située entre les deux parties, des moyens de solidarisation de la housse consistant en un filetage, tandis que ladite housse est percée dans sa région médiane, d'un trou de diamètre correspondant à celui de ladite partie intermédiaire, en sorte de permettre une solidarisation par vissage.

Selon une autre construction particulière du mode de réalisation préférentiel du dispositif pour la désinfection de lit selon l'invention, les deux parties sont prévues solidarisables l'une à l'autre par emboîtement ou vissage, et sont pourvues à cet effet de moyens de solidarisation, lesquels sont également prévus aptes à maintenir la housse par pincement.

Selon une autre caractéristique additionnelle du mode de réalisation préférentiel du dispositif pour la désinfection de lit selon l'invention, l'une ou l'autre des deux parties du moyen de diffusion est pourvue d'un moyen de perforation, apte à perforer la housse en vue d'établir une zone de communication entre lesdites deux parties.

Selon une forme de réalisation préférée du dispositif pour la désinfection de lit selon l'invention, le moyen de diffusion comprend un corps comprenant inférieurement des pieds destinés à reposer sur le matelas en limitant la surface de contact, et intérieurement un moteur électrique entraînant un ventilateur et un compresseur, ce dernier étant associé à un système de délivrance d'une dose de produit nettoyant et/ou désinfectant, et étant prolongé d'une buse de diffusion

Selon une autre caractéristique additionnelle du dispositif pour la désinfection de lit selon l'invention, la housse est réalisée en papier:

Les avantages et les caractéristiques du dispositif pour la désinfection de lit selon l'invention, ressortiront plus clairement de la description qui suit et qui se rapporte au dessin annexé, lequel en représente plusieurs modes de réalisation non limitatifs.

Dans le dessin annexé :
- la figure 1 représente une vue schématique en perspective d'un lit équipé d'un dispositif pour la désinfection de lit selon l'invention.
- la figure 2 représente une vue schématique partielle en coupe partielle et en éclaté du même dispositif.
- la figure 3 représente une vue schématique partielle en élévation d'une variante du dispositif pour la désinfection de lit selon l'invention.

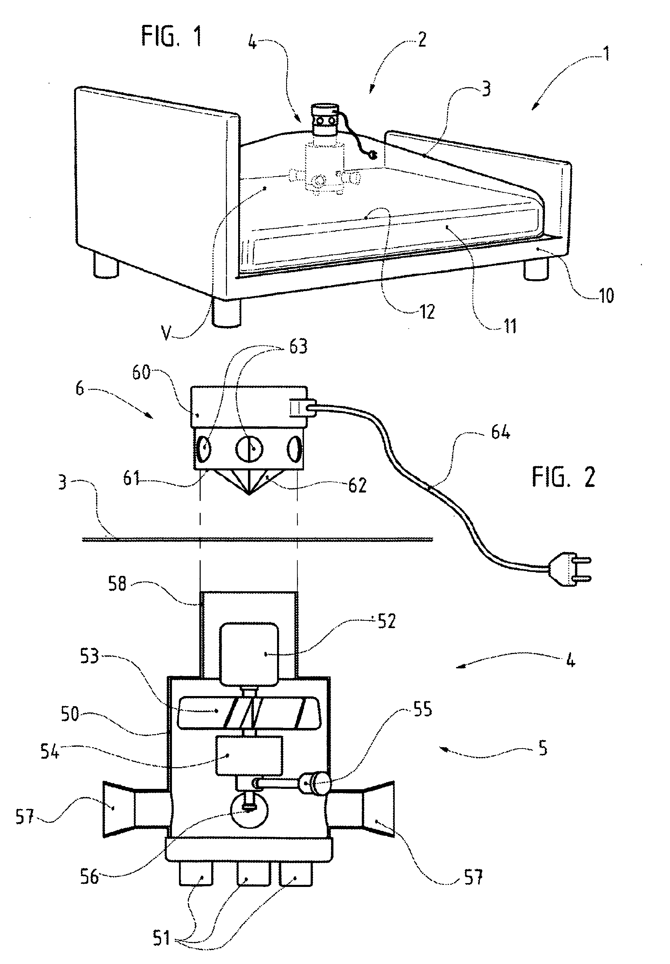

En référence à la figure 1, on peut voir un lit 1 comprenant un châlit 10 et un matelas 11.

Le matelas 11 est surmonté d'un dispositif 2 pour la désinfection de lit selon l'invention lequel comprend une housse 3, et un moyen 4 de diffusion d'un produit de nettoyage et/ou de désinfection.

La housse 3 est adaptée pour couvrir la surface 12 du matelas 11, ainsi que les bords de celui-ci. Elle est suffisamment grande pour permettre d'emprisonner entre elle et le matelas 11 un volume d'air V, et elle est réalisée dans un matériau souple, et de préférence qui peut être déchiré, du papier par exemple.

En référence également à la figure 2, on peut voir que le moyen de diffusion 4 comprend deux parties solidarisables l'une à l'autre de manière réversible, à savoir une partie inférieure 5 et une partie supérieure 6. Ces deux parties 5 et 6 sont destinées à être solidarisées tout en maintenant la housse 3, la partie inférieure 5 demeurant sous cette dernière tandis que la partie supérieure 6 demeure au-dessus.

La partie inférieure 5 comprend une coque 50 de forme globalement cylindrique, comprenant inférieurement des pieds 51 destinés à reposer sur le matelas 11 en limitant la surface de contact, et intérieurement un moteur électrique 52 entraînant un ventilateur 53 et un compresseur 54. Le compresseur 54 est associé à un doseur à capsule 55 de produit nettoyant et/ou désinfectant, et est prolongé d'une buse de diffusion 56.

On notera que le doseur à capsule peut être remplacé par un conteneur équipé d'un moyen de distribution permettant de délivrer une quantité déterminée de produit.

Le moteur électrique 52 est disposé dans le haut de la coque 50, le ventilateur 53 et le compresseur 54 étant disposés successivement en dessous.

La coque 50 comporte de plus dans sa région inférieure, sensiblement au même niveau de la buse 56, et répartis périphériquement, des diffuseurs 57.

La partie supérieure 6 comprend une coque 60 de forme globalement cylindrique, dont l'extrémité inférieure 61 est conçue apte à s'emboîter sur l'extrémité supérieure 58 de la coque 50 qui présente à cet effet une forme complémentaire, tout en maintenant, éventuellement, la housse 3. Elle comporte également intérieurement un moyen de perforation 62, destiné à permettre la perforation de la housse 3 lors de l'emboîtement sur la partie inférieure 5.

La coque 60 présente de plus d'une part des ouvertures 63 constituant des entrées d'air destinées à alimenter le ventilateur 53, et d'autre part un cordon électrique 64 permettant de fournir au moteur 52 l'énergie nécessaire à son fonctionnement, au travers de contacts, non représentés, que comportent les parties inférieure 5 et supérieure 6, et qui sont destinés à être mis en relation lors de l'emboîtement de ces dernières.

Pour mettre en oeuvre le dispositif pour la désinfection de lit selon l'invention, on procède de la manière suivante.

Après enlèvement des draps, la partie inférieure 5 du moyen de diffusion 4 est placée, par ces pieds 51, sur la face supérieure 12 du matelas 11, de préférence au centre, puis on étend pardessus la housse 3 en veillant à bien recouvrir toute la surface du matelas 11, puis on solidarise la partie supérieure 6 du moyen de diffusion 4 sur la partie inférieure 5, en réalisant simultanément la perforation de la housse 3, puis on branche le cordon électrique 64 pour faire fonctionner le moteur 52.

On notera que la solidarisation des deux parties 5 et 6 peut également permettre le coincement entre celles-ci de la housse 3, et donc son immobilisation.

Le ventilateur 53 aspire l'air ambiant au travers des ouvertures 63 et le rejette par les diffuseurs 57, ce qui permet de gonfler la housse 3 et de maintenir le volume V sensiblement constant, l'excédent d'air étant évacué entre le matelas 11 et la housse 3 au niveau des bords de celle-ci.

On notera à ce sujet qu'il est possible de prévoir que la housse 3 comporta des moyens permettant de l'immobiliser sur les bords du matelas 11.

Le moteur 52 actionne simultanément le compresseur 54 qui vaporise le produit nettoyant et/ou désinfectant contenu dans la capsule 55, qui s'échappe par la buse 56 dans le flux d'air et sort par les diffuseurs 57 pour se répandre dans le volume V.

On notera que la housse 3, si elle est réalisée en papier est de préférence pelliculée du côté en regard du matelas 11.

Après un temps prédéfini, de l'ordre de quelques minutes, éventuellement régit par une temporisation, le moteur 52 s'arrête, la housse 3 peut alors être ôtée et jetée, puis le moyen de diffusion 4 est enlevé du matelas 11, lequel peut ensuite être aspiré à l'aide d'un aspirateur.

On notera que, simultanément au traitement du matelas 11, il est possible de réaliser le traitement d'un ou de plusieurs oreillers. Pour cela, on utilise un ou plusieurs éléments supports placés sur le matelas au travers de pieds et comportant un réceptacle ajouré pour recevoir le ou les oreillers, lesquels sont alors comme suspendus au-dessus du matelas 11, dans le volume V, pour pouvoir être traités des deux côtés.

Par ailleurs, de manière avantageuse, la coque 60 renferme un distributeur automatique d'étiquette, qui permet après chaque cycle de nettoyage et/ou de désinfection, de délivrer une étiquette qui peut être fixée sur le matelas, ou sur un oreiller par exemple, et sur laquelle figure la nature de l'opération effectuée ou un marquage permettant d'identifier cette opération, et éventuellement la date de réalisation.

On notera également que de manière avantageuse, le moyen de diffusion 4 comporte, non représentés, des éléments de préhension tels que des poignées, et/ou des moyens d'accrochage en suspension.

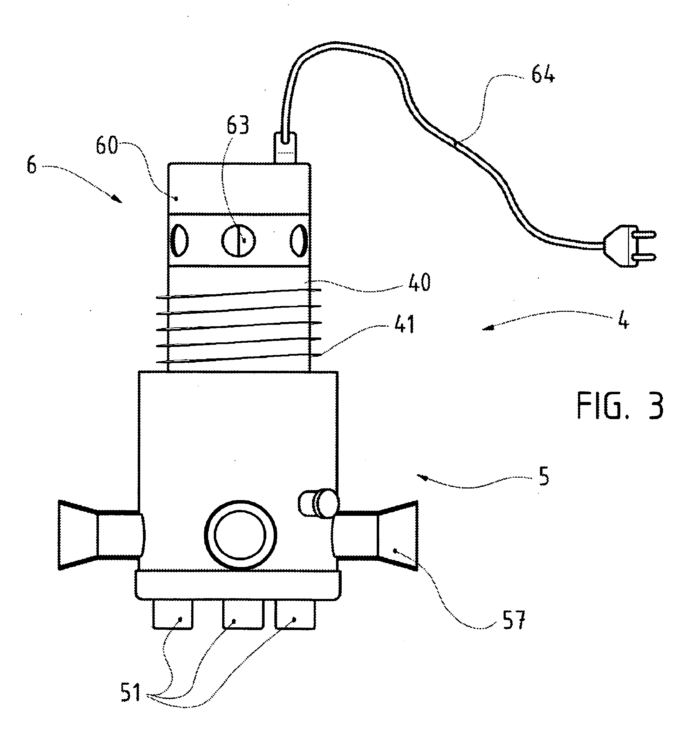

En référence maintenant à la figure 3, on peut voir un autre mode de construction du dispositif 4 pour la désinfection de lit selon l'invention.

Il comporte également une partie inférieure 5 comprenant des pieds 51 et des diffuseurs 57, et une partie supérieure 6 munie d'ouvertures 63 constituant des entrées d'air.

La liaison entre les parties inférieure 5 et supérieure 6, est réalisée au travers d'une partie intermédiaire cylindrique 40, qui présente extérieurement un filetage 41 destiné à permettre la solidarisation par vissage de la housse, non représentée, laquelle comporte à cet effet dans sa région centrale un trou de diamètre correspondant à celui de la partie intermédiaire 40.

## Revendications

1. Dispositif pour la désinfection de lit comprenant
- un moyen de diffusion d'un produit de nettoyage et de désinfection,
- une housse (3) en matière souple apte à recouvrir le matelas (11) dudit lit (1) et éventuellement un ou plusieurs oreillers posés sur ledit matelas (11),
- ledit moyen de diffusion (4) est intercalé, au moins en partie, entre ledit matelas (10) et ladite housse (3), et coopère avec cette dernière en sorte que celle-ci crée une enceinte délimitant la zone de diffusion dudit produit,
- ledit moyen de diffusion (4) comprenant de plus un moyen de ventilation (53) apte à créer un flux garantissant un certain volume d'air (V) entre ladite housse (3) et ledit matelas (11), volume dans lequel est diffusé ledit produit de nettoyage et de désinfection,
charactérisé en ce que le moyen de diffusion (4) comprend deux parties, l'une (5) disposée d'un côte de la housse (3), à savoir en dessous et comprenant des moyens (53, 54, 55, 56, 57) permettant de diffuser le produit, et l'autre disposée de l'autre côté à savoir au-dessus et comprenant des moyens (63) d'aspiration d'air.

2. Dispositif pour la désinfection selon la revendication 1, **caractérisé en ce que** le moyen de diffusion (4) consiste en un moyen de vaporisation (54, 56).

3. Dispositif pour la désinfection selon l'une quelconque des revendications précédentes, **caractérisé en ce que** le moyen de diffusion (4) comporte des moyens (58, 61) aptes à permettre sa solidarisation à la housse (3) dans la région centrale de celle-ci.

4. Dispositif pour la désinfection selon l'une quelconque des revendications précédentes, **caractérisé en ce que** le moyen de diffusion est de forme cylindrique et présente extérieurement, dans une partie intermédiaire (40) située entre les deux parties (5, 6), des moyens de solidarisation de la housse (3) consistant en un filetage (41), tandis que ladite housse (3) est percée dans sa région médiane, d'un trou de diamètre correspondant à celui de ladite partie intermédiaire (41), en sorte de permettre une solidarisation par vissage.

5. Dispositif pour la désinfection selon l'une quelconque des revendications 1 à 3, **caractérisé en ce que** les deux parties (5, 6) sont prévues solidarisables l'une à l'autre par emboîtement ou vissage, et sont pourvues à cet effet de moyens de solidarisations (58, 61), lesquels sont également prévus aptes à maintenir la housse (3).

6. Dispositif pour la désinfection selon la revendication 5, **caractérisé en ce que** l'une ou l'autre des deux parties (5, 6) du moyen de diffusion (4) est pourvue d'un moyen de perforation (62), apte à perforer la housse (3) en vue d'établir une zone de communication entre lesdites deux parties (5, 6).

7. Dispositif pour la désinfection selon l'une quelconque des revendications précédentes, **caractérisé en ce que** le moyen de diffusion (4) comprend un corps (5)comprenant inférieurement des pieds (51) destinés à reposer sur le matelas (11) en limitant la surface de contact, et intérieurement un moteur électrique entraînant (52) un ventilateur (53) et un compresseur (54), ce dernier étant associé à un système (55) de délivrance d'une dose de produit nettoyant et/ou désinfectant, et étant prolongé d'une buse de diffusion (56) disposée en regard de diffuseurs (57) répartis périphériquement.

8. Dispositif pour la désinfection selon l'une quelconque des revendications précédentes, **caractérisé en ce que** la housse (3) est réalisée en papier.

9. Dispositif pour la désinfection selon l'une quelconque des revendications précédentes, **caractérisé en ce qu'**il comporte un ou plusieurs éléments supports placés sur le matelas (11) au travers de pieds et comprenant un réceptacle ajouré pour recevoir un ou plusieurs oreiller.

## Claims

1. Device for disinfecting a bed, comprising
- means for diffusing a cleaning and disinfecting product,
- a cover (3) made out of flexible material, capable of covering the mattress (11) of said bed (1) and eventually one or various pillows placed on said mattress (11),
- said diffusing means (4) is interposed, at least partly, between said mattress (10) and said cover (3), and cooperates with the latter so that it creates an enclosure delimiting the area of diffusion of said product,
- said diffusing means (4) comprising, in addition, ventilating means (53) capable of creating a flow guaranteeing a certain volume of air (V) between said cover (3) and said mattress (11), in which volume said cleaning and disinfecting product is diffused,
wherein the diffusing means (4) comprises two parts, one (5) arranged on one side of the cover (3), i.e. below, and comprising means (53, 54, 55, 56, 57) permitting to diffuse the product, and the other one arranged on the other side, i.e. above, and comprising air-suction means (63).

2. Device for disinfecting according to claim 1, wherein the diffusing means (4) consists of vaporising means (54, 56).

3. Device for disinfecting according to any of the preceding claims, wherein the diffusing means (4) includes means (58, 61) capable of permitting its uniting with the cover (3) in the central region of the latter.

4. Device for disinfecting according to any of the preceding claims, wherein the diffusing means has a cylindrical shape and has, externally, in an intermediate portion (40) located between the two parts (5, 6), means for uniting with the cover (3) consisting of an external thread (41), while said cover (3) is perforated, in its median region, with a hole the diameter of which corresponds to that of said intermediate portion (41), so as to permit their uniting by screwing.

5. Device for disinfecting according to any of claims 1 to 3, wherein the two parts (5, 6) are designed capable of being made integral with each other through encasement or screwing, and are provided to this end with uniting means (58, 61), which are also designed capable of retaining the cover (3).

6. Device for disinfecting according to claim 5, wherein any of the two portions (5, 6) of the diffusing means (4) is provided with perforating means (62) capable of perforating the cover (3) in order to create an area of communication between said two parts (5, 6).

7. Device for disinfecting according to any of the preceding claims, wherein the diffusing means (4) includes a body (5) comprising, at the lower side, feet (51) aimed at resting on the mattress (11) while limiting the contact surface and, internally, an electric motor (52) driving a ventilator (53) and a compressor (54), the latter being associated to a system (55) for delivering a dose of cleaning and/or disinfecting product, and being extended with a diffusing nozzle (56) arranged in front of diffusers (57) distributed over the periphery.

8. Device for disinfecting according to any of the preceding claims, wherein the cover (3) is made out of paper.

9. Device for disinfecting according to any of the preceding claims, wherein the device includes one or several support elements placed on the mattress (11) through feet and comprising an openwork receptacle for receiving one or several pillows.

## Patentansprüche

1. Vorrichtung zum Desinfizieren eines Bettes, umfassend
- Mittel zum Verbreiten eines Reinigungs- und Desinfizierungsprodukts,
- einen Bezug (3) aus biegsamem Material, geeignet, um die Matratze (11) des besagten Bettes (1) zu überziehen und gegebenenfalls ein oder mehrere auf der besagten Matratze (11) gelegten Kopfkissen,
- wobei das besagte Verbreitungsmittel (4) wenigstens zum Teil zwischen der besagten Matratze (10) und dem besagten Bezug (3) zwischengefügt ist, und so mit dem letzteren zusammenwirkt, dass es einen geschlossenen Raum bildet, der den Verbreitungsbereich des besagten Produkt abgrenzt,
- wobei das besagte Verbreitungsmittel (4) außerdem Lüftungsmittel (53) umfasst, die geeignet sind, einen Strom zu schaffen, der ein gewisses Luftvolumen (V) zwischen dem besagten Bezug (3) und der besagten Matratze (11) gewährleistet, in welchem Volumen das besagte Reinigungs- und Desinfizierungsprodukt verbreitet wird,
**dadurch gekennzeichnet, dass** das Verbreitungsmittel (4) zwei Teile umfasst, einen (5), der an einer Seite des Bezugs (3), d.h. unterhalb, angeordnet ist und Mittel (53, 54, 55, 56, 57) umfasst, die es erlauben, das Produkt zu verbreiten, und einen anderen, der an der anderen Seite, d.h. oberhalb, angeordnet ist und Luftansaugmittel (63) umfasst.

2. Vorrichtung zum Desinfizieren nach Anspruch 1, **dadurch gekennzeichnet, dass** das Verbreitungsmittel (4) aus einem Verdunstungsmittel (54, 56) besteht.

3. Vorrichtung zum Desinfizieren nach irgendeinem der vorgehenden Ansprüche, **dadurch gekennzeichnet, dass** das Verbreitungsmittel (4) Mittel (58, 61) umfasst, die geeignet sind, um seine feste Verbindung mit dem Bezug (3) im zentralen Bereich dieses letzteren zu ermöglichen.

4. Vorrichtung zum Desinfizieren nach irgendeinem der vorgehenden Ansprüche, **dadurch gekennzeichnet, dass** das Verbreitungsmittel zylinderförmig ist und außenseitig, in einem zischen den beiden Teilen (5, 6) befindlichen Zwischenteil (40), Mittel zur festen Verbindung mit dem Bezug (3) aufweist, die aus einem Außengewinde (41) bestehen, während der besagte Bezug (3) in seinem Mittelbereich mit einem Loch durchbohrt ist, dessen Durchmesser demjenigen des besagten Mittelteils (41) entspricht, sodaß ihre feste Verbindung durch Schraubverbindung ermöglicht wird.

5. Vorrichtung zum Desinfizieren nach irgendeinem der Ansprüche 1 bis 3, **dadurch gekennzeichnet, dass** beide Teile (5, 6) geeignet vorgesehen sind, um durch Steck- oder Schraubverbindung fest mit einander verbunden zu werden, und zu diesem Zweck mit festen Verbindungsmitteln (58, 61) versehen sind, die ebenfalls geeignet vorgesehen sind, um den Bezug (3) festzuhalten.

6. Vorrichtung zum Desinfizieren nach Anspruch 5, **dadurch gekennzeichnet, dass** irgendeiner der beiden Teile (5, 6) des Verbreitungsmittels (4) mit einem Bohrungsmittel (62) versehen ist, das geeignet ist, um den Bezug (3) zu durchbohren, um einen Verbindungsbereich zwischen den besagten zwei Teilen (5, 6) zu schaffen.

7. Vorrichtung zum Desinfizieren nach irgendeinem der vorgehenden Ansprüche, **dadurch gekennzeichnet, dass** das Verbreitungsmittel (4) einen Körper (5) umfasst, der an der unteren Seite Füßchen (51), die dazu bestimmt sind, auf der Matratze (11) zu ruhen, während sie die Kontaktfläche begrenzen, und innenseitig einen elektrischen Motor (52) umfasst, der einen Ventilator (53) und einen Kompressor (54) antreibt, wobei dieser letztere einem System (55) zum Abgeben einer Dosis des Reinigungs- und Desinfizierungsprodukts zugeordnet ist, und mit einer Verbreitungsdüse (56) verlängert ist, die gegenüber am Umkreis verteilten Verteilern (57) angeordnet ist.

8. Vorrichtung zum Desinfizieren nach irgendeinem der vorgehenden Ansprüche, **dadurch gekennzeichnet, dass** der Bezug (3) aus Papier hergestellt ist.

9. Vorrichtung zum Desinfizieren nach irgendeinem der vorgehenden Ansprüche, **dadurch gekennzeichnet, dass** sie ein oder mehrere Tragelemente umfassst, die über Füßchen auf der Matratze (11) gesetzt sind und einen durchlochten Behälter zum Aufnehmen von einem oder mehreren Kopfkissen umfassen.
